# EUROPEAN PATENT APPLICATION

(11) **EP 4 154 855 A1**
(43) Date of publication of application: **29.03.2023**
(21) Application number: 20936094.0
(22) Date of filing: 20.05.2020
(51) Int. Cl.: A61F 13/532, A61F 13/49, A61F 13/535

(54) **DISPOSABLE DIAPER**

(71) Applicant: Kao Corporation, Chuo-ku Tokyo 103-8210 (JP)
(72) Inventor: FUKUDA, Yuko, Haga-gun, Tochigi 321-3497 (JP); TSUGE, Kyoko, Haga-gun, Tochigi 321-3497 (JP)
(74) Representative: Vossius & Partner Patentanwälte Rechtsanwälte mbB
(86) International application number: PCT/JP2020/020022
(87) International publication number: WO 2021/234881

(57) **Abstract**

A diaper 1 includes an absorbent member (4). In a crotch portion (C), the absorbent member (4) includes: a central absorbent member (4C) extending in the longitudinal direction; and a pair of side absorbent members (4S, 4S) respectively located on both widthwise sides of the central absorbent member, the side absorbent members configured to stand up from a skin-facing surface side of the central absorbent member in a worn state. In a reference swollen state, compression load to compress a test piece of the diaper (1) until the test piece's thickness becomes 30 mm is 7 N or less in the crotch portion, the test piece being obtained by folding the diaper (1) along the longitudinal direction in a manner that the diaper's width is divided equally in two. In the reference swollen state, the thickness of the side absorbent member (4S) is 10 mm or less, or compression load to compress the side absorbent member until the thickness thereof becomes 10 mm is 4.5 cN or less.

## Description

### Technical Field

The present invention relates to a disposable diaper.

### Background Art

A typical absorbent article, such as a disposable diaper, is provided with an absorbent member including an absorbent core, as a liquid-absorbing section for absorbing body fluid such as urine. From the viewpoint of improving leakage preventability and fittability to the wearer's skin, there have been proposed absorbent articles including an absorbent core having a plurality of regions. The plurality of regions are divided by depressions, such as slits or grooves, or by varying the thickness, basis weight, etc., of the respective regions.

### Citation List

### Patent Literature

Patent Literature 1: JP 2006-247363A
Patent Literature 2: WO 2014/200794A1

### Summary of Invention

The present invention relates to a disposable diaper including a topsheet, a backsheet, and an absorbent member arranged between the topsheet and the backsheet, the absorbent member including an absorbent core, the disposable diaper having a longitudinal direction and a width direction orthogonal to the longitudinal direction, the disposable diaper having a rear portion to be arranged on a wearer's rear side when worn, a front portion to be arranged on the wearer's front side when worn, and a crotch portion located between the front portion and the rear portion.

Preferably, in the crotch portion, the absorbent member includes: a central absorbent member extending in the longitudinal direction; and a pair of side absorbent members respectively located on both widthwise sides of the central absorbent member, the side absorbent members configured to stand up from a skin-facing surface side of the central absorbent member in a worn state.

Preferably, in a reference swollen state achieved by injecting 160 g of artificial urine to an injection point located 7 cm away toward the front portion side in the longitudinal direction from a center position of the disposable diaper, which is at a center in the longitudinal direction and at a center in the width direction, and leaving the disposable diaper to stand for 5 minutes, compression load to compress a test piece of the disposable diaper until the test piece's thickness becomes 30 mm is 7 N or less in the crotch portion, the test piece being formed by folding the disposable diaper along the longitudinal direction in a manner that the disposable diaper's width is divided equally in two.

Preferably, in the reference swollen state, the side absorbent member's thickness is 10 mm or less, or compression load to compress the side absorbent member until the thickness thereof becomes 10 mm is 4.5 N or less.

### Brief Description of Drawings

[Fig. 1] Fig. 1 is a perspective view illustrating an embodiment of a disposable diaper of the present invention.
[Fig. 2] Fig. 2 is a developed plan view schematically illustrating a skin-facing surface side of the disposable diaper illustrated in Fig. 1 in a spread-open and stretched state.
[Fig. 3] Fig. 3 is a cross-sectional view taken along line II-II illustrated in Fig. 2.
[Fig. 4] Fig. 4(a) is a cross-sectional view taken along the width direction of an absorbent member in a state before absorbing liquid, and Fig. 4(b) is a cross-sectional view taken along the width direction of the absorbent member in a reference swollen state.
[Fig. 5] Fig. 5 is a cross-sectional view taken along the diaper's width direction, illustrating, together with the wearer's crotch region, the diaper's crotch portion in a swollen state and having been deformed in a worn state.
[Fig. 6] Fig. 6 is an explanatory diagram for illustrating a method for measuring 15-mm-width compression load of the central absorbent member.
[Fig. 7] Fig. 7 is an explanatory diagram for illustrating a method for measuring 30-mm compression load.
[Fig. 8] Fig. 8 illustrates diagrams corresponding to Fig. 4, illustrating another embodiment of an absorbent member according to the present invention.
[Fig. 9] Figs. 9(a) and 9(b) are diagrams corresponding to Fig. 4, illustrating yet another embodiment of an absorbent member according to the present invention.
[Fig. 10] Figs. 10(a) to 10(c) are diagrams corresponding to Fig. 4, illustrating yet another embodiment of an absorbent member according to the present invention.
[Fig. 11] Fig. 11 illustrates diagrams corresponding to Fig. 4, illustrating yet another embodiment of an absorbent member according to the present invention.
[Fig. 12] Fig. 12 is a plan view illustrating an embodiment of bending-guide portions according to the present invention.
[Fig. 13] Fig. 13 is a diagram corresponding to Fig. 12, illustrating another embodiment of bending-guide portions according to the present invention.
[Fig. 14] Fig. 14 is a plan view illustrating another embodiment of an absorbent core according to the present invention.

### Description of Embodiments

Applicant has previously proposed, as an absorbent article including an absorbent core having a plurality of regions, a disposable diaper provided with an absorbent member including a central absorbent member, a pair of side absorbent members, and gaps formed between these absorbent members, the disposable diaper being configured such that, in the crotch portion, both lateral sides of an absorbent assembly including an absorbent core are capable of standing up (Patent Literature 1).

Patent Literature 2 discloses an absorbent article including one or more area(s) substantially free of absorbent material, wherein a top side of a core wrap, which is for enclosing the absorbent material, is attached to a bottom side of the core wrap through the one or more area(s). When the absorbent material swells, the core wrap forms one or more channel(s) along the area(s).

Absorbent cores typically include absorbent material such as an absorbent polymer, and hence swell upon absorption of body fluid such as urine. If the absorbent core swells excessively, the entire disposable diaper may become too bulky. This may cause the disposable diaper to abut against the wearer's femoral regions and restrict the wearer's movement. Particularly, if the movement around the femoral region is restricted, walking may be inhibited. This may become a hinderance when a toddler etc. is learning to walk or when an elderly person etc. is performing walking rehabilitation. Unfortunately, Patent Literatures 1 and 2 do not disclose techniques for facilitating movement around the wearer's femoral regions even when the absorbent core has swollen.

The present invention relates to a disposable diaper capable of eliminating restrictions in the wearer's movement caused by swelling/expansion of the absorbent core.

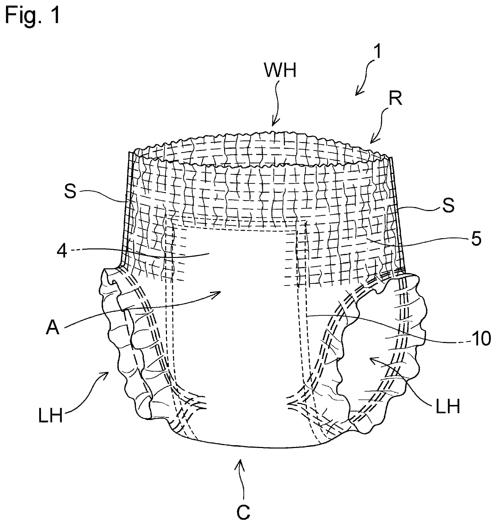

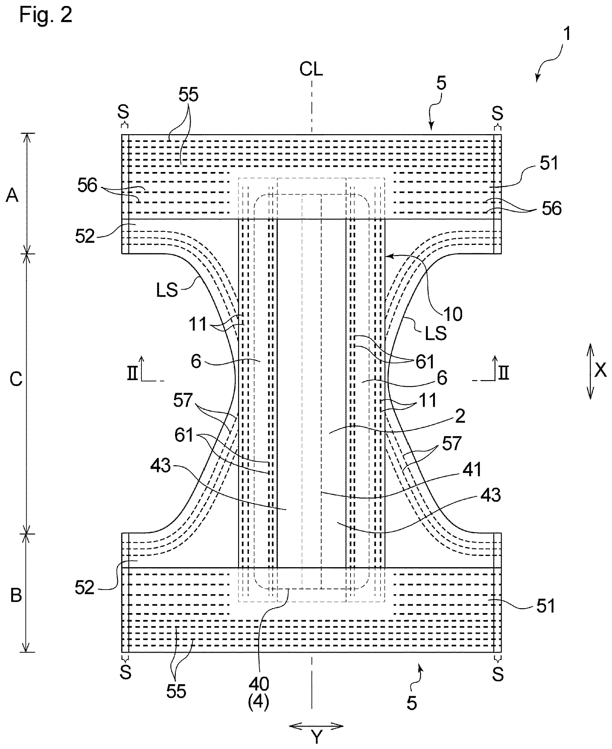

The present invention is described below according to preferred embodiments thereof with reference to the drawings. Figs. 1 and 2 illustrate a pull-on disposable diaper 1 (also referred to hereinafter simply as "diaper 1") which is an embodiment of an absorbent article of the present invention. As illustrated in Fig. 1, the diaper 1 includes: a front portion A to be arranged on the wearer's front side in a state where the diaper 1 is worn; a rear portion B to be arranged on the wearer's rear side in a state where the diaper 1 is worn; and a crotch portion C located between the front portion and the rear portion. The diaper 1 has a longitudinal direction X corresponding to the wearer's front-rear direction-i.e., corresponding to a direction extending from the front portion A to the rear portion B via the crotch portion C-and a width direction Y orthogonal to the longitudinal direction. As illustrated in Fig. 2, the diaper 1 is formed so as to have left-right symmetry with respect to a longitudinal center line CL that extends along the longitudinal direction X and that divides the diaper 1 equally in two in the width direction Y

The diaper 1 includes, in a central portion in the width direction Y, an absorbent assembly 10 including an absorbent member 4, and the diaper also includes an outer cover 5 provided on the non-skin-facing surface side of the absorbent assembly 10-i.e., provided on a side farther from the wearer's body than the absorbent assembly 10.

The absorbent member 4 extends between the front portion A and the rear portion B, and includes: a liquid-retentive absorbent core 40; and a core-wrap sheet 48 that comes into contact with the outer surface of the absorbent core 40 and covers the same.

In the outer cover 5, a pair of side seals S, S, a waist opening WH through which the wearer's body passes, and a pair of leg openings LH (only one is illustrated in the figure) through which the wearer's legs pass are formed by joining together the outer cover 5's both lateral side edge portions, which extend along the longitudinal direction X, in the respective front portion A and the rear portion B by using a known joining means such as an adhesive, e.g. a hot-melt adhesive, heat sealing, ultrasonic sealing, etc.

In this Description, the "skin-facing surface" is the surface of the absorbent article such as a disposable diaper, as well as its constituent members (e.g., the topsheet), that faces the wearer's skin side when the absorbent article is worn-i.e., the side relatively closer to the wearer's skin-whereas the "non-skin-facing surface" is the surface of the absorbent article, as well as its constituent members, that faces the opposite side from the wearer's skin side when the absorbent article is worn-i.e., the side relatively farther from the wearer's skin. Herein, "when worn" and "worn state/wearing state" refer to a state in which the absorbent article is maintained in its ordinary, proper wearing/attachment position-i.e., the correct wearing/attachment position of the absorbent article.

The absorbent assembly 10 has a rectangular shape in a planar view in a spread-open and stretched state of the diaper 1, as illustrated in Fig. 2. The absorbent assembly 10 extends in the longitudinal direction X from the front portion A to the rear portion B, and is arranged in the outer cover 5's central portion in the width direction Y in a manner that the absorbent assembly's longitudinal direction matches the longitudinal direction X of the diaper 1 in its spread-open and stretched state, and is joined to the outer cover 5 by an adhesive.

Herein, a "spread-open and stretched state" of the diaper 1 refers to a state in which the side seals S of the diaper 1 are torn apart to bring the diaper in a spread-out (developed) state, and the elastic members in various parts of the spread-out diaper 1 are stretched so that the diaper is spread to its designed size (size when the diaper is spread out in a planar manner in a state where the effect of the elastic members is completely eliminated).

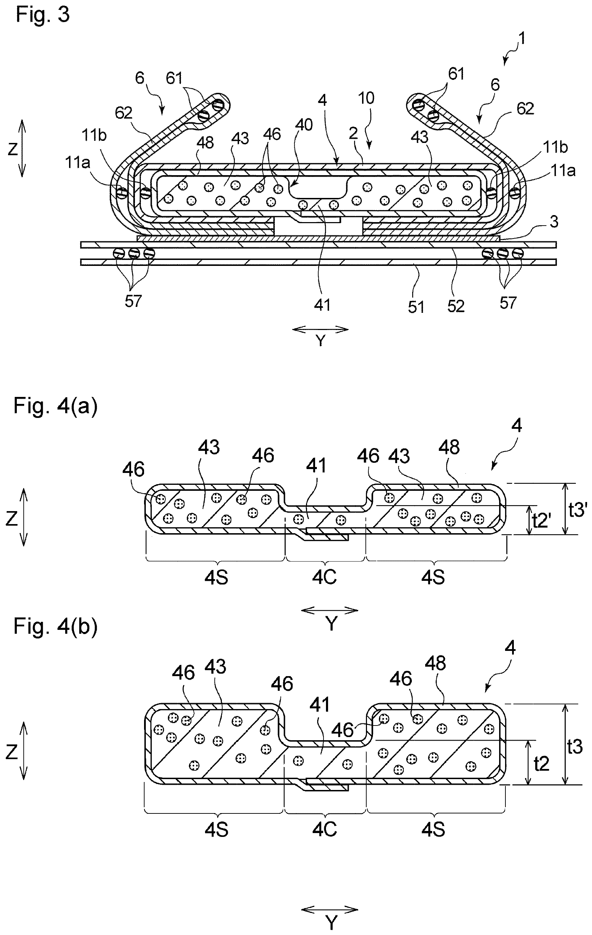

As illustrated in Fig. 3, the absorbent assembly 10 includes: a liquid-permeable topsheet 2 forming the skin-facing surface; a liquid-impermeable or sparingly liquid-permeable or water-repellent backsheet 3 forming the non-skin-facing surface; and a liquid-retentive absorbent member 4 interposed between the two sheets 21, 22. The two sheets and the absorbent member are integrated by a known joining means such as an adhesive.

In the present embodiment, the topsheet 2 covers the skin-facing surface side of the absorbent member 4, and the topsheet's both side end portions are folded back toward the non-skin-facing surface side of the absorbent member 4.

For the topsheet 2 and the backsheet 3, it is possible to use, without particular limitation, one of various materials conventionally used in this type of absorbent article. For example, for the topsheet 2, it is possible to use one of various nonwoven fabrics or a porous film. For the backsheet 3, for example, it is possible to use a resin film or a laminate of a resin film and a nonwoven fabric.

In a planar view as illustrated in Fig. 2, the absorbent member 4 has a rectangular shape that is long in the longitudinal direction X, and extends along the longitudinal direction X from the front portion A to the rear portion B. In the present embodiment, the respective positions of the absorbent member 4's both end portions in the longitudinal direction X substantially match the respective positions of the absorbent assembly 10's both end portions in the longitudinal direction X.

As illustrated in Fig. 2, a pair of leak-proof cuffs 6, 6 is provided respectively on both lateral sides along the longitudinal direction X on the skin-facing surface of the absorbent assembly 10. Each leak-proof cuff is formed by a liquid-resistant/water-repellent and air-permeable leak-proof-cuff-forming sheet 62. Each leak-proof cuff 6 includes one or more thread-shaped leak-proof-cuff-forming elastic members 61 provided along the longitudinal direction X in a stretched state. Each leak-proof cuff 6 stands up at least in the crotch portion C by the contraction of the elastic members 61 when the diaper 1 is worn. The leak-proof cuffs 6 are each formed by folding the continuous leak-proof-cuff-forming sheet 62 in two along its longitudinal direction, then fixing the leak-proof-cuff-forming elastic members 61 in their stretched state between opposing portions of the leak-proof-cuff-forming sheet, and joining together the opposing portions of the sheet.

The outer cover 5 forms the outer shape of the diaper 1 in its spread-open and stretched state as illustrated in Fig. 2. The peripheral edge of the outer cover 5 forms the contour line of the diaper 1 in the aforementioned state-i.e., forms the contour line in each of the front portion A, the crotch portion C, and the rear portion B. As illustrated in Fig. 2, in the crotch portion C, the outer cover 5's both lateral side edge portions, which extend along the outer cover's longitudinal direction X, are curved in an arc-shape protruding toward the central portion in the width direction Y, and thus, the outer cover 5's central region in the longitudinal direction X is narrowed inwardly in the width direction Y

As illustrated in Figs. 2 and 3, the outer cover 5 includes: an outer sheet 51 that forms the outer surface, i.e., the non-skin-facing surface, of the diaper 1; an inner sheet 52 provided on the skin-facing surface side of the outer sheet 51; and a plurality of elastic members 55, 56, 57 provided between the two sheets 51, 52.

In the present embodiment, as illustrated in Fig. 2, the outer cover 5 includes: hip elastic members 55 located more outward, in the longitudinal direction X, than the absorbent assembly 10; and elastic members 56 located more inward than the absorbent assembly 10's both end portions in the longitudinal direction X. The elastic members 56 which are provided in positions overlapping the absorbent assembly 10 in the longitudinal direction X may be finely cut/separated or otherwise treated so as not to exhibit elasticity/extensibility in a region overlapping the absorbent assembly 10 in the thickness direction. The outer cover 5 includes, in each of the front portion A and the rear portion B, a plurality of thread-shaped or band-shaped elastic members 55, 56 arranged in the width direction Y in a stretched state, and the elastic members 55, 56 are arranged intermittently with predetermined intervals therebetween in the longitudinal direction X. By providing the elastic members 55, 56 in a state exhibiting elasticity/extensibility, the opening edge of the waist opening WH is provided with annular waist gathers (creases) which are substantially continuous over the entire circumference.

Referring to the outer cover 5, as illustrated in Figs. 1 and 2, in each leg edge portion LS forming the opening edge of the respective leg opening LH, at least one thread-shaped or band-shaped leg elastic member 57 for forming leg gathers is provided in a stretched state. Thus, annular leg gathers are formed substantially continuously over the entire circumference of the opening edge of each leg opening LH. The elastic members 55, 56, 57 in the outer cover 5 are sandwiched and fixed between the outer sheet 51 and the inner sheet 52, which constitute the outer cover 5, by a joining means such as an adhesive.

As illustrated in Fig. 3, the absorbent assembly 10 of the present embodiment includes elastic members 11a, 11b extending along the longitudinal direction X on both lateral sides, in the width direction Y, of the absorbent member 4. More specifically, the absorbent assembly includes, on the outside of each of the absorbent member 4's lateral sides in the width direction Y: a leak-proof-cuff-side elastic member 11a fixed in a stretched state between the leak-proof-cuff-forming sheet 62; and an absorbent-member-side elastic member 11b fixed in a stretched state between the absorbent member 4 and the topsheet 2. The end portions of these elastic members 11a, 11b are fixed in the front portion A and the rear portion B with an adhesive etc.

In the diaper 1 of the present embodiment, in addition to the contraction of the leak-proof-cuff-forming elastic members 61 along the longitudinal direction X, the leak-proof-cuff-side elastic member 11a and the absorbent-member-side elastic member 11b also contract in the longitudinal direction X. Thus, both lateral sides of the absorbent assembly 10 along the longitudinal direction X can stand up toward the skin-facing surface side more easily than the section located between the lateral sides. This configuration also contributes to making the pair of side absorbent members 4S, 4S of the absorbent member 4 stand up from the skin-facing surface side of the central absorbent member 4C.

As illustrated in Fig. 2, in the crotch portion C, the absorbent member 4 includes: a central absorbent member 4C extending in the longitudinal direction; and a pair of side absorbent members 4S, 4S respectively located on both widthwise sides, in the width direction Y, of the central absorbent member 4C, the side absorbent members configured to stand up from the skin-facing surface side of the central absorbent member 4C in a worn state.

The side absorbent members 4S, 4S are capable of standing up as described above by, for example, configurations (i1) and (i2) of the absorbent member 4. The configurations (i1) and (i2) will be described further below.

The side absorbent members 4S may be configured to stand up from the central absorbent member 4C in the later-described reference swollen state, or may be configured to stand up from the central absorbent member 4C even before the absorbent member 4 absorbs liquid.

For the sake of convenience of explanation, in Fig. 3, the side absorbent members 4S are illustrated in a state where they are not standing up.

The absorbent member 4 includes an absorbent core 40, and a core-wrap sheet 48 covering the surface of the absorbent core 40. The absorbent core 40 includes absorbent materials, such as an absorbent polymer 46, fiber material (not illustrated), etc. As illustrated in Fig. 2, the absorbent core 40 has a shape that is long in one direction. At least in the crotch portion C, the absorbent core 40 has a central region 41 in the central absorbent member 4C and side regions 43 in the respective side absorbent members 4S. Stated differently, the absorbent core 40 includes: a pair of side regions 43, 43 extending along the longitudinal direction X in the crotch portion C; and a central region 41 located between the pair of side regions 43, 43.

In the absorbent core 40 of the present embodiment, the central region 41 and the side regions 43 are continuous in the width direction Y. In the absorbent member 4 of the present embodiment, a single core-wrap sheet 48 collectively covers the central region 41 and the side regions 43.

In a diaper 1 into which excretion fluid, such as urine, has been excreted, the absorbent core 40 swells as a result of absorbing excreted fluid. A model of a diaper in a state where excretion fluid has been excreted can be prepared according to the following method. First, an unused diaper (product) is brought into a spread-open and stretched state. Then, a mark (also referred to as "first mark") is made with an oil-based pen on the skin-facing surface of the topsheet 2 at the center position of the diaper 1, the center position being at the center in the longitudinal direction X and at the center in the width direction Y. Then, another mark is made at a position 7 cm away toward the front portion A side in the longitudinal direction X from the center position marked with the first mark, and the position marked with this other mark is employed as the injection point for injecting artificial urine. Next, a section forming the diaper 1's waist opening WH is gripped, and the diaper 1's crotch portion C is made to hang down such that the center position with the first mark is located at the lowest position. This state is referred to as "hanging-down state". In this hanging-down state, 160 g of artificial urine is injected to the injection point at an injection rate of 5 g/second. A tube pump can be used for this injection. Then, after injection of artificial urine, the diaper 1 is left to stand in the hanging-down state for 5 minutes. This state is referred to as "reference swollen state". Note that 160 g of artificial urine is equivalent to the amount of three to four times of urination by an infant/toddler.

The composition of the artificial urine is as follows: 1.94 mass% urea, 0.7954 mass% sodium chloride, 0.11058 mass% magnesium sulfate (heptahydrate), 0.06208 mass% calcium chloride (dihydrate), 0.19788 mass% potassium sulfate, 0.0035 mass% polyoxyethylene lauryl ether, and ion-exchanged water (balance).

In the reference swollen state, the diaper 1 has the following physical properties (1) and (2), or the following physical properties (1) and (3).
(1) In the reference swollen state, as regards a test piece 1a obtained by folding the diaper 1 along the longitudinal direction X in a manner that the diaper 1's width is divided equally in two, compression load to compress the test piece until the test piece's thickness becomes 30 mm (referred to hereinbelow as "30-mm compression load") is 7 N or less in the crotch portion C.
(2) In the side absorbent member 4S in the reference swollen state, the side absorbent member 4S's thickness t3 (see Fig. 4(b)) is 10 mm or less.
(3) In the reference swollen state, compression load to compress the side absorbent member 4S until the side absorbent member 4S's thickness t3 becomes 10 mm (referred to hereinafter as "10-mm compression load") is 4.5 N or less.

The preferred configurations of the diaper 1 described below are applicable to both embodiments having the physical properties (1) and (2) and embodiments having the physical properties (1) and (3), unless specifically stated otherwise.

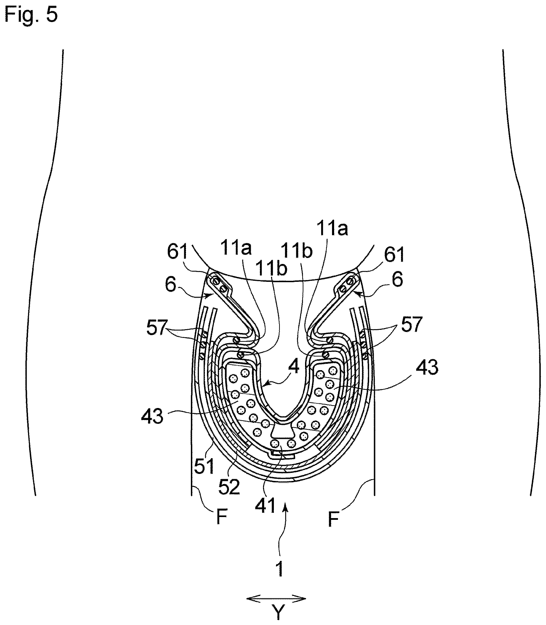

The effects of the diaper 1 in the reference swollen state are described below, taking an embodiment having the physical properties (1) and (2) as an example. In the diaper 1, the central absorbent member 4C constitutes the bottom portion, whereas the pair of side absorbent member 4S, 4S, located on respective lateral sides of the central absorbent member 4C in the width direction Y, stands up from the skin-facing surface side of the central absorbent member 4C. Thus, the entire crotch portion C is deformed in a U shape in a cross-sectional view along the width direction Y (see Fig. 5). In the test piece 1a in the reference swollen state, the 30-mm compression load of the crotch portion C is 7 N or less, and hence, the diaper 1's crotch portion C can easily deform into the U shape. The reason for employing 30-mm compression load as an index is based on the following findings. That is, when a wearer wears a diaper having a width of 30 mm in the crotch portion C, the diaper hardly interferes with the wearer's walking motion. It was also found that the amount of load for compressing the test piece 1a in the reference swollen state to a thickness of 30 mm greatly affects the wearer's ease of walking.

Inventors have also found that the width at the wearer's crotch section is approximately 30 mm, with no significant difference between toddlers and adults. The side absorbent member 4S's thickness t3 in the aforementioned property (2) has therefore been defined based on this finding. By providing the diaper with the aforementioned physical property (2), the crotch portion C after deformation in the aforementioned U shape becomes easily fittable in a compact manner to the wearer's crotch section, as illustrated in Fig. 5.

Thus, in a worn state and in the reference swollen state, the diaper 1 having the aforementioned physical properties (1) and (2) easily deforms into a U shape in a cross-sectional view of the crotch portion C taken along the width direction Y and is thus easily fittable in a compact manner in the width direction Y. Thus, even in a swollen state after absorbing excreted fluid, the entire crotch portion C is suppressed from getting bulky in the width direction Y. Also, the diaper 1 is less likely to abut against the wearer's femoral regions F, F, and the wearer's movement around the femoral region F is less likely to be restricted. As described above, even in a swollen state, the diaper 1 is less likely to hinder walking motion. Thus, the wearer of the diaper 1 can walk easily.

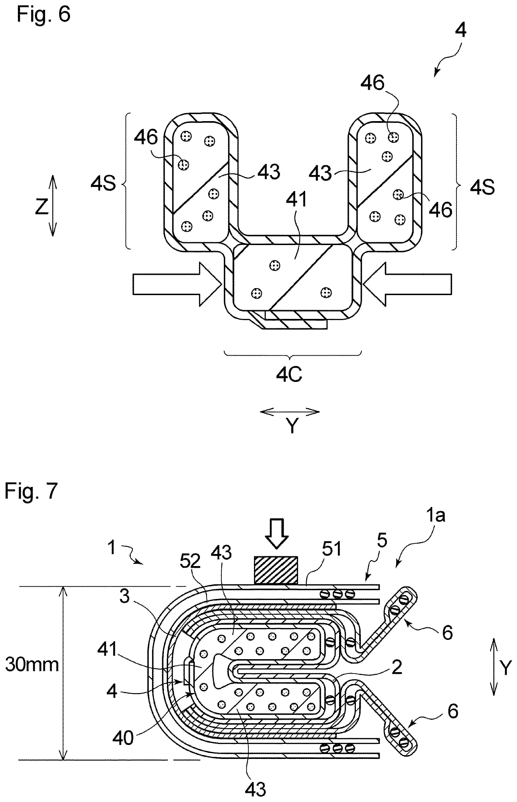

The test piece 1a in the reference swollen state can be employed as a model of the diaper 1 located at the wearer's crotch section and having absorbed liquid such as urine (see Fig. 7).

From the viewpoint of making the diaper 1 deform more easily in the width direction Y and thereby further suppressing the diaper 1 in the reference swollen state from applying pressure to the femoral regions, it is preferable that, as regards the aforementioned physical property (1), the 30-mm compression load of the test piece 1a in the crotch portion C is preferably 0.1 N or greater, more preferably 0.15 N or greater, and preferably 7 N or less, more preferably 6.5 N or less, and preferably from 0.1 to 7 N, more preferably from 0.15 to 6.5 N.

As regards the aforementioned physical property (1), the method for measuring the 30-mm compression load of the test piece 1a is as follows. First, the diaper is brought into a spread-open and stretched state. Then, the diaper is folded along the central line CL in the longitudinal direction so as to divide the width of the diaper equally in two, and this is employed as a test piece 1a. This test piece 1a is placed on a horizontal place in a manner that there are no creases or folds and that the longitudinal direction X matches the horizontal direction. A rectangular acrylic plate, being 5 cm wide and 15 cm long and weighing 28.7 g, is placed at the test piece 1a's center position in the longitudinal direction X (referred to hereinafter as "first measurement position") and also at a position located 2.5 cm away from the center position toward the front portion A side (referred to hereinafter as "second measurement position"). At this time, the acrylic plate is placed such that the acrylic plate's width matches the diaper's longitudinal direction X and such that the center of the plate's width matches the first measurement position or the second measurement position. Each acrylic plate is moved downward at a compression rate of 100 mm/minute, to compress the test piece 1a until its thickness becomes 30 mm (see Fig. 7). A material tester (e.g., Autograph AG-X from Shimadzu Corporation) is used for the compression. The compression load for when the diaper's thickness has become 30 mm is measured at each of the first measurement position and the second measurement position, and the mean value of the two points is considered to be the 30-mm compression load.

In cases where the aforementioned physical property (2) is satisfied, from the viewpoint of making the deformed crotch portion C more compact, the side absorbent member 4S's thickness t3 (see Fig. 4(b)) in the reference swollen state is preferably 3 mm or greater and more preferably 5 mm or greater in cases where the thickness is 10 mm or less, and is more preferably 8 mm or less, and is preferably from 3 to 10 mm, more preferably from 5 to 8 mm.

The thicknesses at the various parts of the absorbent member 4 or the absorbent core 40 can be measured according to the following method.

The absorbent member 4 is taken out from the diaper 1 in the reference swollen state. The absorbent member 4 is placed on a horizontal place in a manner that there are no creases or folds and that the skin-facing surface faces upward in the vertical direction. Next, using a thickness gage Peacock Dial Upright Gauges R5-C (from Ozaki Mfg. Co., Ltd.), the thickness of the central absorbent member 4C and that of the side absorbent member 4S are measured under a load of 5 cN/cm². More specifically, the thickness of a section where the central region 41 is present in the central absorbent member 4C, or the thickness of a section where the side region 43 is present in the side absorbent member 4S, is measured. At this time, the thickness is measured by arranging, between the tip end portion of the thickness gage and the sample, a plate (an acrylic plate having a thickness of approximately 5 mm) having a circular or square planar-view shape and whose size has been adjusted such that the load is 5 cN/cm². At the time of measurement, in cases where the thickness of the central region 41 is small and the core-wrap sheet 48 in the central region 41 rises up from the absorbent core 40, the absorbent member is cut and separated along the boundaries between the central region 41 and the respective side regions 43, and the thickness of the central absorbent member 4C is measured in the cut-out section in a manner that the absorbent core 40 and the core-wrap sheet 48 within that section are in contact with one another. This measurement is performed at five arbitrary sites within a central range in the longitudinal direction for both the section where the central region 41 is present in the central absorbent member 4C and the section where the side region 43 is present in the side absorbent member 4S. The mean value of the measurement values for the five sites is considered to be the thickness of the central absorbent member 4C or the thickness of the side absorbent member 4S. A value found by subtracting the thickness of the core-wrap sheet 48 from the thickness of the central absorbent member 4C is considered to be the thickness of the central region 41, and a value found by subtracting the thickness of the core-wrap sheet 48 from the thickness of the side absorbent member 4S is considered to be the thickness of the side region 43. The aforementioned "central range in the longitudinal direction" is a range that passes through the center of the absorbent member 4 in the longitudinal direction X and that is within 5 cm on both sides in the longitudinal direction X from a lateral line extending in the width direction Y-i.e., a range within 5 cm in the longitudinal direction X toward both the front portion A side and the rear portion B side from the aforementioned lateral line.

In cases of removing a constituent member, such as the absorbent member etc., from the diaper, the constituent member is removed by first spraying the diaper with a cold spray to solidify the adhesive bonding the constituent members together, and then peeling off the constituent member from the other constituent members. This removal method is applicable in common to other measurement methods described in the present Description, unless specifically stated otherwise.

Effects of the diaper 1 having the aforementioned physical property (1) and the aforementioned physical property (3), instead of the aforementioned physical property (2), will be described. In the diaper 1 having the physical properties (1) and (3), since the compression load of the aforementioned property (3) is 4.5 N or less, the softness of the crotch portion C can be enhanced and the crotch portion C can be deformed easily, even if the crotch portion C in the reference swollen state abuts against the wearer's femoral regions F, F (see Fig. 5). Thus, the crotch portion C in the reference swollen state is less likely to apply pressure to the femoral regions F, F, and the wearer's movement around the femoral region F is less likely to be restricted. Thus, also with the diaper 1 having the physical properties (1) and (3), the crotch portion C in the reference swollen state can deform easily, and thereby, the crotch portion C is less likely to hinder walking motion, and the wearer of the diaper 1 can walk easily.

From the viewpoint of further suppressing the crotch portion C from applying pressure to the femoral regions F, it is preferable that the 10-mm compression load of the side absorbent member 4S in the aforementioned physical property (3) is preferably 0.1 N or greater, and preferably 4.5 N or less, more preferably 4 N or less, and preferably from 0.1 cN to 4.5 N, more preferably from 0.1 to 4 N.

It is preferable that the 10-mm compression load in both the side absorbent members 4S, 4S is within the aforementioned range.

The method for measuring the 10-mm compression load is as follows. First, the absorbent member 4 is removed from a diaper in the reference swollen state, and the absorbent member 4 is placed on a horizontal place in a manner that there are no creases or folds and that its skin-facing surface faces upward in the vertical direction. Next, using a material tester (e.g., Autograph AG-X from Shimadzu Corporation) equipped with a 2-cm-dia. rod-shaped compression test jig, a section where the absorbent core 40 is present in the side absorbent member 4S is compressed, and the compression load to compress that section until its thickness becomes 10 mm is measured. This measurement is performed at three arbitrary sites in a section where the absorbent core 40 is present in the side absorbent member 4S, and the mean value thereof is considered to be the 10-mm compression load.

From the viewpoint of making the deformed crotch portion C more compact, it is preferable that the central absorbent member 4C's thickness t2 and the side absorbent member 4S's thickness t3 in the reference swollen state are within the following ranges.

In the reference swollen state, it is preferable that the ratio t3/t2 of t3 to t2, where t2 (see Fig. 4(b)) is the central absorbent member 4C's thickness and t3 (see Fig. 4(b)) is the side absorbent member 4S's thickness, is preferably 1.3 or greater, more preferably 2 or greater, and preferably 40 or less, more preferably 30 or less, and preferably from 1.3 to 40, more preferably from 2 to 30.

The thickness t2 of the central absorbent member 4C in the reference swollen state refers to the thickness of a section (central region 41) where the absorbent core 40 is present in the central absorbent member 4C in the reference swollen state. The thickness t3 of the side absorbent member 4S in the reference swollen state refers to the thickness of a section (side region 43) where the absorbent core 40 is present in the side absorbent member 4S in the reference swollen state.

The thickness t2 (see Fig. 4(b)) of the central absorbent member 4C in the reference swollen state is preferably 0.5 mm or greater, more preferably 1 mm or greater, and preferably 30 mm or less, more preferably 25 mm or less, and preferably from 0.5 to 30 mm, more preferably from 1 to 25 mm.

In cases where the aforementioned physical property (3) is satisfied, it is preferable that the thickness t3 (see Fig. 4(b)) of the side absorbent member 4S in the reference swollen state is preferably 10 mm or greater, more preferably 15 mm or greater, and preferably 30 mm or less, more preferably 25 mm or less, and preferably from 10 to 30 mm, more preferably from 15 to 25 mm.

In the absorbent member 4 of the present embodiment, in a worn state and in the reference swollen state, the boundary section between the central absorbent member 4C and each side absorbent member 4S serves as an axis of flexure, and each of the side absorbent members 4S is configured to stand up from the skin-facing surface side of the central absorbent member 4C. The aforementioned axis of flexure can be formed, for example, by providing the absorbent member 4 with at least one of the following configurations (i1) and (i2). The absorbent member 4 may have both the following configurations (i1) and (i2).
(i1) The central absorbent member 4C's thickness and the side absorbent member 4S's thickness are different from one another either before absorbing liquid or in the reference swollen state.
(i2) A bending-guide portion is formed between the central absorbent member 4C and the side absorbent member 4S.

By providing the absorbent member 4 with at least one of the aforementioned configurations (i1) and (i2), an axis of flexure will be formed at the boundary section between the central absorbent member 4C and each side absorbent member 4S in a worn state and in the reference swollen state. The axis of flexure is formed at the boundary section between the central absorbent member 4C and each side absorbent member 4S by at least one of the aforementioned configurations (i1) and (i2), and thus, the central absorbent member 4C and each side absorbent member 4S in the absorbent member 4 are divided by the aforementioned configuration (i1) or (i2).

The aforementioned axis of flexure increases the flexibility of the crotch portion C and thereby enables the crotch portion C of the absorbent member 4 to deform easily into the aforementioned U shape. Thus, the resistance at the time of compressing the crotch portion C inwardly in the width direction Y can be reduced. Stated differently, in terms of the aforementioned physical property (1), forming the aforementioned axis of flexure makes it easier to control the 30-mm compression load of the test piece 1a in the crotch portion C to be 7 N or less.

In cases where the absorbent member 4 has the aforementioned configuration (i1), the difference in thickness between the central absorbent member 4C and the side absorbent member 4S either before absorbing liquid or in the reference swollen state forms the axis of flexure for bending the absorbent member 4, with the boundary section between the central absorbent member 4C and each side absorbent member 4S serving as a base point.

From the viewpoint of making the crotch portion C deform into a U shape that is more compact in the width direction Y and also reliably securing the absorption capacity of the absorbent core 40, it is preferable that, in cases where the absorbent member 4 has the aforementioned configuration (i1), the side absorbent member 4S's thickness is greater than the central absorbent member 4C's thickness in a state before the absorbent member 4 absorbs liquid.

From the same viewpoint, in cases where the absorbent member 4 has the aforementioned configuration (i1), it is preferable that the central absorbent member 4C's thickness and the side absorbent member 4S's thickness in a state before the absorbent member 4 absorbs liquid are within the following ranges. The thickness of each section of the absorbent member 4 before absorbing liquid refers to the thickness of a section where the absorbent core 40 is present in the absorbent member.

In a state before the absorbent member 4 absorbs liquid, the ratio t3'/t2' of t3' to t2', where t2' (see Fig. 4(a)) is the central absorbent member 4C's thickness and t3' (see Fig. 4(a)) is the side absorbent member 4S's thickness, is preferably 1.3 or greater, more preferably 2 or greater, and preferably 40 or less, more preferably 30 or less, and preferably from 1.3 to 40, more preferably from 2 to 30.

The central absorbent member 4C's thickness t2' (see Fig. 4(a)) before the absorbent member 4 absorbs liquid is preferably 0.5 mm or greater, more preferably 1 mm or greater, and preferably 15 mm or less, more preferably 10 mm or less, and preferably from 0.5 to 15 mm, more preferably from 1 to 10 mm.

The side absorbent member 4S's thickness t3' (see Fig. 4(a)) before the absorbent member 4 absorbs liquid is preferably 10 mm or greater, more preferably 15 mm or greater, and preferably 30 mm or less, more preferably 25 mm or less, and preferably from 10 to 30 mm, more preferably from 15 to 25 mm, on condition that it is greater than the central absorbent member 4C's thickness t2' before the absorbent member 4 absorbs liquid.

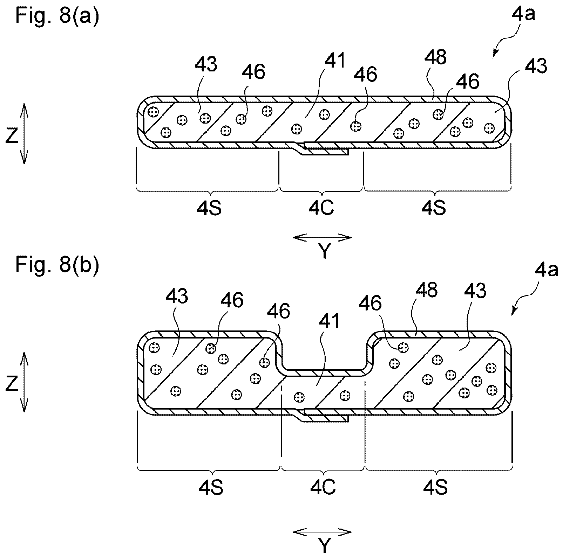

As illustrated in Figs. 4(a) and 4(b), in the absorbent member 4 of the present embodiment, the side absorbent member 4S's thickness is greater than the central absorbent member 4C's thickness both before absorbing liquid and in the reference swollen state. Stated differently, the side absorbent member 4S's thickness is greater than the central absorbent member 4C's thickness before and after assuming the reference swollen state. Instead, as illustrated in Fig. 8(a), in the absorbent member 4, the side absorbent members 4S and the central absorbent member 4C may have the same thickness in a state before the absorbent member 4 absorbs liquid. In this case, when the absorbent member 4 is in the reference swollen state, the side absorbent member 4S's thickness and the central absorbent member 4C's thickness may be different from one another (see Fig. 8(b)), or the side absorbent members 4S and the central absorbent member 4C may have the same thickness (not illustrated).

An example of a form in which the absorbent member 4 has the aforementioned configuration (i1) may include a form in which the content ratio of the absorbent polymer is different between the central absorbent member 4C and the side absorbent member 4S. For example, in the reference swollen state, the member, among the central absorbent member 4C and the side absorbent members 4S, having a higher content ratio of the absorbent polymer will have a greater thickness, thus creating a difference in thickness between the central absorbent member 4C and the side absorbent members 4S. As a result, an axis of flexure will be formed at the boundary section between the central absorbent member 4C and each side absorbent member 4S.

Further, the content ratio of the absorbent polymer in each side absorbent member 4S also affects the compression load and the thickness of the side absorbent member 4S in the reference swollen state. Thus, this also contributes to achieving the physical properties (2) and (3).

The "content ratio of the absorbent polymer in the central absorbent member 4C" refers to the ratio of the mass of the absorbent polymer included in the central absorbent member 4C with respect to the mass of the central absorbent member 4C including the absorbent core and the core-wrap sheet. The "content ratio of the absorbent polymer in the side absorbent member 4S" refers to the ratio of the mass of the absorbent polymer included in the side absorbent member 4S with respect to the mass of the side absorbent member 4S including the absorbent core and the core-wrap sheet.

As illustrated in Fig. 3, the absorbent core 40 of the present embodiment contains an absorbent polymer 46. From the viewpoint of making the side absorbent member 4S's thickness greater than the central absorbent member 4C's thickness in the reference swollen state and allowing the side absorbent member 4S to stand up more easily, in cases where the content ratio of the absorbent polymer is different between the central absorbent member 4C and the side absorbent member 4S, it is preferable that the side absorbent member 4S has a higher content ratio of the absorbent polymer 46 than the central absorbent member 4C.

From the same viewpoint, it is preferable that the ratio F2/F1 of F2 to F1, where F1 is the content ratio of the absorbent polymer 46 in the central absorbent member 4C and F2 is the content ratio of the absorbent polymer 46 in the side absorbent member 4S, is preferably 1.3 or greater, more preferably 1.5 or greater, and preferably 3 or less, more preferably 2.5 or less, and preferably from 1.3 to 3, more preferably from 1.5 to 2.5.

The content ratio F1 of the absorbent polymer 46 in the central absorbent member 4C is preferably 30% or greater, more preferably 35% or greater, and preferably 70% or less, more preferably 65% or less, and preferably from 30% to 70%, more preferably from 35% to 65%.

The content ratio F2 of the absorbent polymer 46 in the side absorbent member 4S is preferably 40% or greater, more preferably 45% or greater, and preferably 100% or less, more preferably 90% or less, and preferably from 40% to 100%, more preferably from 45% to 90%, on condition that the content ratio of the absorbent polymer 46 is higher than that of the central absorbent member 4C. This configuration is preferable in terms that the aforementioned physical properties (2) and (3) can be achieved easily.

An example of a form in which the absorbent member 4 has the aforementioned configuration (i1) may include a form in which the basis weight of the absorbent core 40 is different between the central absorbent member 4C and the side absorbent member 4S. For example, in cases where the density of the absorbent core 40 in the central absorbent member 4C and in the side absorbent member 4S is the same whereas the basis weight of the absorbent core 40 is different between the central absorbent member 4C and the side absorbent member 4S, the member having a higher basis weight will have a greater thickness, thus creating a difference in thickness between the central absorbent member 4C and the side absorbent members 4S. As a result, an axis of flexure will be formed at the boundary section between the central absorbent member 4C and each side absorbent member 4S.

Further, the basis weight in the side absorbent member 4S also affects the compression load and the thickness of the side absorbent member 4S in the reference swollen state. Thus, this also contributes to achieving the physical properties (2) and (3).

From the viewpoint of further suppressing the crotch portion C from getting bulky in the width direction Y, it is preferable that, in cases where the basis weight is different between the central absorbent member 4C and the side absorbent member 4S, the absorbent core 40's basis weight in the side absorbent member 4S is greater than the absorbent core 40's basis weight in the central absorbent member 4C.

From the same viewpoint, it is preferable that the absorbent core 40's basis weight in the side absorbent member 4S and that in the central absorbent member 4C are within the following ranges. The basis weight is measured in a state before absorbing liquid. In the following description, the preferred basis weight of the absorbent core 40 in the side absorbent member 4S refers to the basis weight of a single side absorbent member 4S, and not the total basis weight for the pair of side absorbent members 4S, 4S.

The ratio B2/B1 of B2 to B1, where B1 is the absorbent core 40's basis weight in the central absorbent member 4C and B2 is the absorbent core 40's basis weight in the side absorbent member 4S, is preferably 1.1 or greater, more preferably 1.5 or greater, and preferably 3 or less, more preferably 2.5 or less, and preferably from 1.1 to 3, more preferably from 1.5 to 2.5.

The absorbent core 40's basis weight B1 in the central absorbent member 4C is preferably 1 g/m² or greater, more preferably 5 g/m² or greater, and preferably 200 g/m² or less, more preferably 100 g/m² or less, and preferably from 1 to 200 g/m², more preferably from 5 to 100 g/m².

The absorbent core 40's basis weight B2 in the side absorbent member 4S is preferably 80 g/m² or greater, more preferably 150 g/m² or greater, and preferably 700 g/m² or less, more preferably 600 g/m² or less, and preferably from 80 to 700 g/m², more preferably from 150 to 600 g/m², on condition that it is greater than the absorbent core 40's basis weight in the central absorbent member 4C. This configuration is preferable in terms that the physical properties (2) and (3) can be achieved easily.

In cases where the absorbent member 4 has the aforementioned configuration (i2), the bending-guide portion will serve as the axis of flexure for causing each of the side absorbent members 4S to stand up. In this case, the central absorbent member 4C and each side absorbent member 4S in the absorbent member 4 are divided by the respective bending-guide portion.

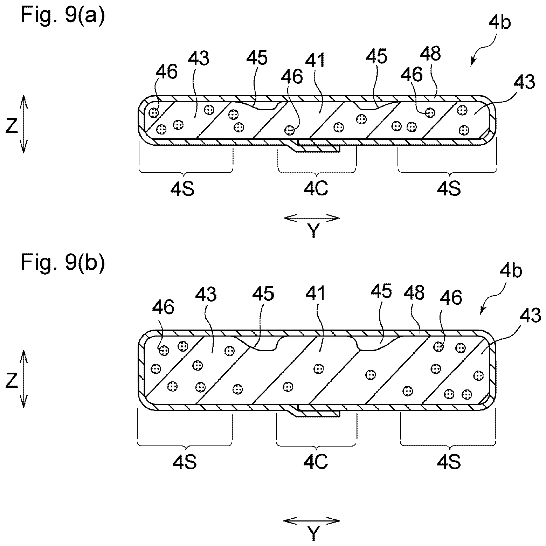

Absorbent members having the aforementioned configuration (i2) will be described as other embodiments of disposable diapers of the present invention, with reference to the drawings. The explanation on the foregoing embodiment applies as appropriate to the other embodiments, unless there are contradictions. In Figs. 9 to 14, constituent elements that are the same as those in the foregoing embodiment are accompanied by the same reference signs as in the foregoing embodiment.

Each of the absorbent members 4b, 4c, 4d illustrated in Figs. 9 to 11 includes bending-guide portions 45 formed between the central absorbent member 4C and the respective side absorbent members 4S in the crotch portion C.

Each of these absorbent members 4 includes a pair of bending-guide portions 45, 45. The central absorbent member 4C is located between the pair of bending-guide portions 45, 45 in the width direction Y, and the side absorbent members 4S are located outside the respective bending-guide portions 45 in the width direction Y. The pair of side absorbent members 4S is configured to stand up from the skin-facing surface side of the central absorbent member 4C, employing the bending-guide portions 45 as base points.

In such configurations wherein the absorbent core 40 includes a pair of bending-guide portions 45, 45-i.e., configurations wherein the central region 41 and the side regions 43 are not continuous in the width direction Y on the absorbent core 40's skin-facing surface or non-skin-facing surface, the central absorbent member 4C and the side absorbent members 4S are divided by the pair of bending-guide portions 45, 45.

In a configuration wherein the depression or through hole forming the bending-guide portion 45 is not clear, the width in the crotch portion C of the absorbent member 4 may be divided equally in three into three regions, and the region located in the center among the three regions may be specified as the central absorbent member 4C, whereas the regions located on both sides of the centrally-located region may be specified as the side absorbent members 4S. In cases where the width of the crotch portion C varies along the longitudinal direction X, the width of the crotch portion C is to be divided equally in three at the position of the narrowest portion where the width becomes the shortest.

The absorbent member 4b illustrated in Figs. 9(a) and 9(b) includes bending-guide portions 45 respectively constituted by a depression. The bending-guide portions 45 are formed in the skin-facing surface of the absorbent core 40. The absorbent core 40 illustrated in Fig. 9 has a central region 41 between the depressions, which respectively form the bending-guide portions 45, in the width direction Y, and has side regions 43 outside the respective depressions in the width direction Y.

The depression may be a recessed portion formed by embossing etc., or may be a depression formed by a non-fiber-stack portion where there is no material forming the absorbent core, and a fiber-stack portion in which material forming the absorbent core is present.

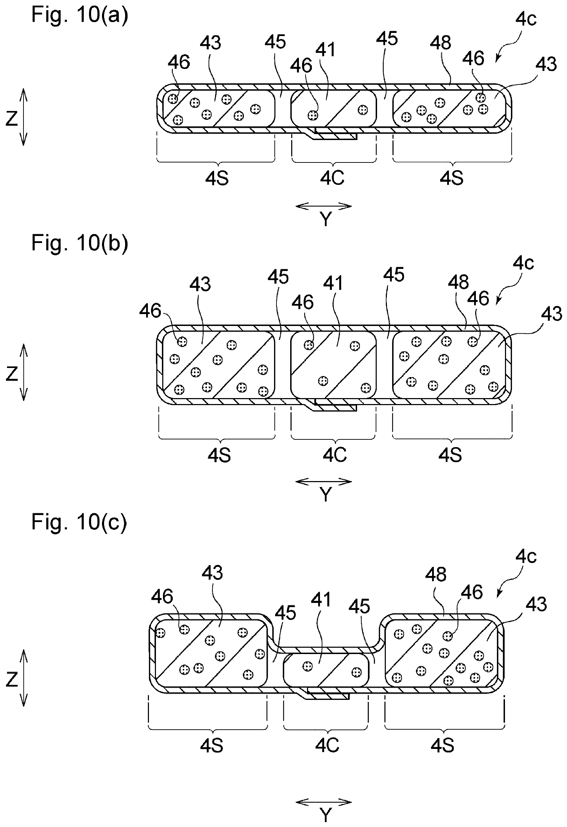

The absorbent member 4c illustrated in Fig. 10 includes bending-guide portions 45 respectively constituted by a through hole penetrating the absorbent core 40. The bending-guide portion 45 may be a slit having a width of 30 mm or less.

In the absorbent member 4 illustrated in Fig. 10, the core-wrap sheet 48 located on the absorbent core 40's skin-facing surface side and the core-wrap sheet located on the non-skin-facing surface side are not joined together within each bending-guide portion 45, which is constituted by a through hole.

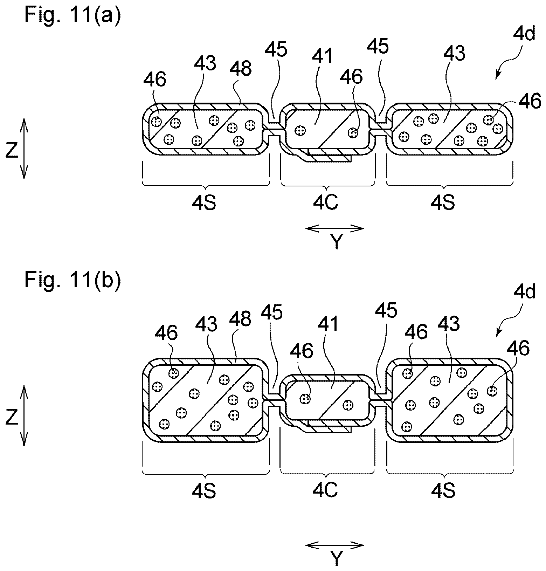

The absorbent member 4d illustrated in Fig. 11 includes bending-guide portions 45 respectively constituted by a through hole, similar to the absorbent member illustrated in Fig. 10. In contrast, the core-wrap sheet 48 located on the absorbent core 40's skin-facing surface side and the core-wrap sheet located on the non-skin-facing surface side are joined together within each bending-guide portion 45. With this configuration, the absorbent member 4 can deform into the aforementioned U shape more easily in the reference swollen state.

In the absorbent member 4 including the bending-guide portions 45, the side absorbent members 4S and the central absorbent member 4C may have the same thickness in a state before the absorbent member 4 absorbs liquid (see Figs. 9(a), 10(a), and 11(a)), or the side absorbent members 4S and the central absorbent member 4C may have different thicknesses (not illustrated). In the absorbent member 4 in the reference swollen state, the side absorbent members 4S and the central absorbent member 4C may have the same thickness (see Figs. 9(b) and 10(b)), or the side absorbent members 4S and the central absorbent member 4C may have different thicknesses (see Figs. 10(c) and 11(b)).

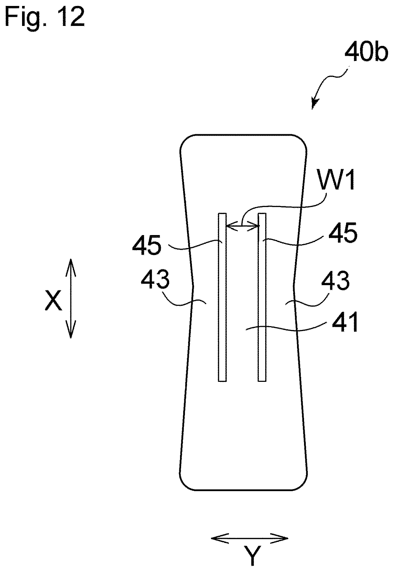

Figs. 12 to 14 illustrate plan views of absorbent cores according to the present invention. The following explanation on the embodiments illustrated in Figs. 12 to 14 applies to a state of the absorbent core before absorbing liquid, unless specifically stated otherwise as "reference swollen state".

In a planar view of the absorbent core, the bending-guide portions 45 may be formed rectilinearly as illustrated in Fig. 12. The bending-guide portion 45 illustrated in Fig. 12 extends along the longitudinal direction of the absorbent core 40b, and has the same width over the entire length of the bending-guide portion 45.

Stated differently, the pair of bending-guide portions 45, 45 extends rectilinearly along the longitudinal direction X of the absorbent core 40b, and thereby, the width of the central region 41 is constant along the longitudinal direction of the absorbent core 40b. With this configuration, the lateral side edges of the diaper 1, which has assumed the reference swollen state and whose cross-sectional shape has been deformed into the U shape, is less likely to abut against the wearer's femoral regions, and thus, the range of motion of the wearer's femoral regions can be widened. As a result, movements, such as large rotation of the femoral region about the femoral axis, can be facilitated.

From the viewpoint of further widening the range of motion of the wearer's femoral regions, it is preferable that the width W1 (see Fig. 12) of the central region 41 in the reference swollen state is preferably 8 mm or greater, more preferably 10 mm or greater, and preferably 35 mm or less, more preferably 30 mm or less, and preferably from 8 to 35 mm, more preferably from 10 to 30 mm.

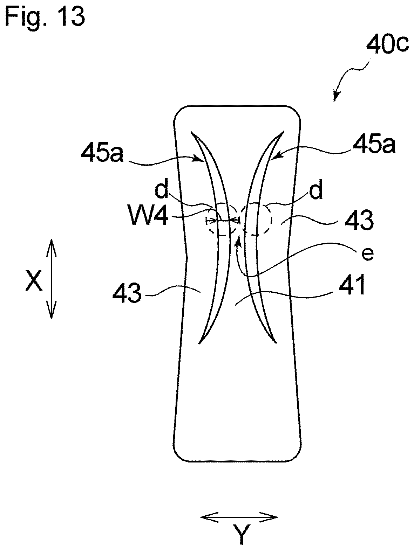

As illustrated in Fig. 13, the bending-guide portions 45 may be formed curvilinearly. The central region 41 illustrated in Fig. 13 has a width that varies along the longitudinal direction X. More specifically, each of the bending-guide portions 45a, 45a, which extend along the longitudinal direction X, is formed curvilinearly, and, in the vicinity of the center in the longitudinal direction X, each bending-guide portion is curved inwardly in the width direction Y.

In the configuration illustrated in Fig. 13, the central region 41 formed by the contour of the bending-guide portions 45a has a narrowed portion e where both lateral side edges extending along the longitudinal direction X are narrowed inwardly in the width direction Y, the narrowed portion e being located more toward the front portion A side than the center in the longitudinal direction X. With this configuration, a section of the absorbent core 40 more toward the front portion A side than the center in the longitudinal direction X can be easily bent into the aforementioned U shape, thereby further facilitating the motion of moving the femoral region forward.

In the absorbent core 40, the bending-guide portion 45 may have the same width along the longitudinal direction X as illustrated in Fig. 12, or may have a varying width along the longitudinal direction X as illustrated in Fig. 13.

For example, the width of each bending-guide portion 45a illustrated in Fig. 13 varies along the longitudinal direction X, and each bending-guide portion has a maximum width portion d where the width becomes the greatest, the maximum width portion d being located more toward the front portion A side than the center in the longitudinal direction X. With this configuration, the aforementioned front portion A side of the absorbent core 40 can be bent into the aforementioned U shape more easily, thereby further facilitating the motion of moving the femoral region forward.

From the viewpoint of achieving the aforementioned effect more reliably, it is preferable that the width W4 (see Fig. 13) of the bending-guide portion 45a's maximum width portion d is preferably 10 mm or greater, more preferably 12 mm or greater, and preferably 30 mm or less, more preferably 20 mm or less, and preferably from 10 to 30 mm, more preferably from 12 to 20 mm.

The shape of the contour of the bending-guide portion 45 may include rectilinear portions as illustrated in Fig. 12, or may include curvilinear portions as illustrated in Fig. 13.

Alternatively, the shape of the contour of the bending-guide portion 45 may include both rectilinear portions and curvilinear portions.

Next, a planar-view shape of the absorbent core 40 in the crotch portion C will be described. In the absorbent core 40, both lateral side edges along the longitudinal direction X may be rectilinear and parallel to the longitudinal direction X over the entire length of the absorbent core (see Fig. 2).

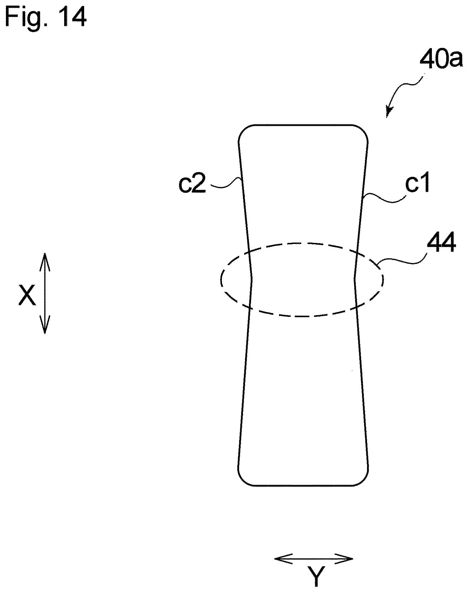

Instead, the absorbent core may have a width that varies along the longitudinal direction X. For example, the absorbent core 40a illustrated in Fig. 14 has a narrowed portion 44 where both lateral side edges c1, c2 of the absorbent core extending along the longitudinal direction X are narrowed inwardly in the width direction Y In Fig. 14, the central region 41 and the pair of side regions 43, 43 are omitted from illustration.

From the viewpoint of further facilitating the motion of moving the femoral region forward and further facilitating walking motion, it is preferable that the absorbent core 40a has the narrowed portion 44 located more toward the front portion A side than the center in the longitudinal direction X.

From the same viewpoint, it is preferable that the narrowed portion 44 is located in the crotch portion C of the diaper 1, and more preferably located more toward the front portion A side than the center of the crotch portion C in the longitudinal direction X.

The absorbent core 40a having the narrowed portion 44 may be included in an absorbent member having the aforementioned configuration (i1), or may be included in an absorbent member having the aforementioned configuration (i2).

The absorbent core 40 may have a single-layer structure constituted by a layer made from absorbent material (also referred to as core-forming material), or may have a layered structure in which a plurality of such layers are stacked.

The number of layers forming the central region 41 may be smaller than the number of layers forming the side regions 43.

The central absorbent member 4C may include a fiber-stack portion in which core-forming material is present as in the aforementioned single-layer structure or layered structure, and may also include a non-fiber-stack portion where there is no core-forming material.

The absorbent cores 40 according to the foregoing embodiments can be manufactured using a known fiber-stacking device. The fiber-stacking device is a device that typically includes a rotary drum having an accumulation depression in the outer circumferential surface thereof, wherein: core-forming materials are supplied to the drum's outer circumferential surface in a dispersed and airborne state while rotating the rotary drum, and the core-forming materials are stacked in the accumulation depression by suction from the bottom surface of the accumulation depression; and the fiber stack within the accumulation depression is then released from the accumulation depression by suction by a suction means arranged in opposition to the accumulation depression, and is transferred onto the suction means.

In the thus-configured fiber-stacking device, a portion of the air-permeable bottom surface of the accumulation depression is made air-impermeable or sparingly air-permeable by, for example, arranging an air-impermeable member or a sparingly air-permeable member on a portion of the bottom surface. In this accumulation depression, the core-forming materials are less prone to get stacked on the air-impermeable or sparingly air-permeable portion at the time of stacking the core-forming materials, and thus, the amount of core-forming materials stacked on the air-impermeable or sparingly air-permeable portion becomes smaller compared to other sections of the bottom surface. By providing such air-impermeable or sparingly air-permeable portions, it is possible to form an absorbent member in which the basis weight of core-forming materials is made different between the central absorbent member 4C and the side absorbent members 4S, and/or an absorbent member including bending-guide portions. That is, the aforementioned absorbent member 4 having the aforementioned configuration (i1) or (i2) can be obtained by using such a fiber-stacking device having a rotary drum in which a portion of the bottom surface of the accumulation depression is made air-impermeable or sparingly air-permeable, to thereby adjust the fiber-stacking amount of core-forming materials in the accumulation depression.

Materials for forming the various parts of the disposable diapers of the foregoing embodiments are described below.

As for absorbent materials primarily constituting the absorbent core 40, any material conventionally used as a material for an absorbent member in this type of absorbent article may be used without particular limitation, with examples including wood pulp, hydrophilized synthetic fibers, absorbent polymers, etc. A typical form of the absorbent core may be, for example, a fiber aggregate of hydrophilic fibers such as wood pulp, or a member in which absorbent polymer particles are retained in the aforementioned fiber aggregate.

In the diaper 1, the core-wrap sheet 48 covers the entire region of the absorbent core's skin-facing surface and non-skin-facing surface. The core-wrap sheet 48 may be a single continuous sheet, or may include a single skin-side core-wrap sheet which covers the absorbent core 40's skin-facing surface and a single non-skin-side core-wrap sheet which is separate from the skin-side core-wrap sheet and covers the absorbent core 40's non-skin-facing surface. For the core-wrap sheet 48, it is possible to use, for example, a liquid-permeable sheet such as paper, one of various types of nonwoven fabrics, a porous film, etc.

The absorbent core and the core-wrap sheet may be joined together by a known joining means such as a hot-melt adhesive.

For sheets constituting the outer cover 5, such as the outer sheet 51, the inner sheet 52, etc., it is possible to use nonwoven fabrics made by various manufacturing methods, with examples including spunbond nonwoven fabrics, air-through nonwoven fabrics, spun-laced nonwoven fabrics, heat-rolled nonwoven fabrics, meltblown nonwoven fabrics, or laminated nonwoven fabrics of the above.

For the various elastic members 11a, 11b, 61, 55, 56, 57 described above, it is possible to use, for example, synthetic rubber such as styrene-butadiene, butadiene, isoprene or neoprene, natural rubber, EVA, extensible polyolefins, or polyurethane. As for the shapes of the elastic members, it is possible to preferably use thread-form members (rubber threads) having a rectangular, square, circular, elliptic, or polygonal cross-sectional shape, or band-form members (rubber bands), or multifilament-type thread-form members.

The present invention has been described above according to preferred embodiments thereof, but the present invention is not limited to the foregoing embodiments and can be modified as appropriate. Also, the foregoing embodiments may be employed in combination.

For example, as illustrated in Fig. 2, the aforementioned diaper 1 is a pull-on disposable diaper including an outer cover 5 having an hourglass-like shape in which the front portion A, the crotch portion C and the rear portion B are continuous. The diaper may, however, be a separate-type pull-on disposable diaper wherein the outer cover 5 is divided into a front-side outer cover, a rear-side outer cover and a crotch outer cover as separate members. The disposable diaper of the present invention is not limited to a pull-on disposable diaper, and may be an open-type disposable diaper. Further, the disposable diaper of the present invention may be a disposable diaper either for toddlers or adults.

In the foregoing embodiments, the diaper 1 includes leak-proof-cuff-forming elastic members 61 and elastic members 11a, 11b located outside the absorbent member 4 in the width direction Y. However, it will suffice if the diaper includes either one of these elastic members 61, 11a, 11b, or includes two or more of these elastic members.

In the foregoing embodiments, each leak-proof cuff 6 is formed by folding the leak-proof-cuff-forming sheet 62 in two. The leak-proof cuff, however, may be formed such that the leak-proof-cuff-forming sheet 62 is folded back only in the section where the leak-proof-cuff-forming elastic members 61 are to be fixed, and other sections are constituted by a single sheet.

In relation to the foregoing embodiments of the present invention, the present invention further discloses the following disposable diapers.
{1} A disposable diaper comprising a topsheet, a backsheet, and an absorbent member arranged between the topsheet and the backsheet,
   the absorbent member including an absorbent core,
   the disposable diaper having a longitudinal direction and a width direction orthogonal to the longitudinal direction,
   the disposable diaper having a rear portion to be arranged on a wearer's rear side when worn, a front portion to be arranged on the wearer's front side when worn, and a crotch portion located between the front portion and the rear portion, wherein:
      in the crotch portion, the absorbent member includes
      a central absorbent member extending in the longitudinal direction, and
      a pair of side absorbent members respectively located on both widthwise sides of the central absorbent member, the side absorbent members being configured to stand up from a skin-facing surface side of the central absorbent member in a worn state;
      in a reference swollen state achieved by injecting 160 g of artificial urine to an injection point located 7 cm away toward the front portion side in the longitudinal direction from a center position of the disposable diaper, which is at a center in the longitudinal direction and at a center in the width direction, and leaving the disposable diaper to stand for 5 minutes, compression load to compress a test piece of the disposable diaper until the test piece's thickness becomes 30 mm is 7 N or less in the crotch portion, the test piece being formed by folding the disposable diaper along the longitudinal direction in a manner that the disposable diaper's width is divided equally in two; and
   in the reference swollen state, the side absorbent member's thickness is 10 mm or less, or compression load to compress the side absorbent member until the thickness thereof becomes 10 mm is 4.5 N or less.
{2} The disposable diaper as set forth in clause {1}, wherein, in the reference swollen state, compression load to compress the test piece until the thickness thereof becomes 30 mm is from 0.1 to 7 N, preferably from 0.15 to 6.5 N.
{3} The disposable diaper as set forth in clause {1} or {2}, wherein, in the side absorbent member in the reference swollen state, the side absorbent member's thickness t3 is from 3 to 10 mm, preferably from 5 to 8 mm.
{4} The disposable diaper as set forth in clause {1} or {2}, wherein, in the reference swollen state, compression load to compress the side absorbent member until the side absorbent member's thickness t3 becomes 10 mm is from 0.1 cN to 4.5 N, preferably from 0.1 to 4 N.
{5} The disposable diaper as set forth in any one of clauses {1} to {4}, wherein, in a state before the absorbent member absorbs liquid, the side absorbent member's thickness is greater than the central absorbent member's thickness.
{6} The disposable diaper as set forth in clause {5}, wherein, in a state before the absorbent member absorbs liquid, the ratio t3'/t2' of the side absorbent member's maximum thickness t3' to the central absorbent member's maximum thickness t2' is from 1.3 to 40, preferably from 2 to 30.
{7} The disposable diaper as set forth in clause {5} or {6}, wherein, in the reference swollen state, the ratio t3/t2 of the side absorbent member's thickness t3 to the central absorbent member's thickness t2 is from 1.3 to 40, preferably from 2 to 30.
{8} The disposable diaper as set forth in any one of clauses {1} to {7}, wherein:
   the absorbent core includes an absorbent polymer; and
   the side absorbent member has a higher content ratio of the absorbent polymer than the central absorbent member.
{9} The disposable diaper as set forth in clause {8}, wherein the content ratio of the absorbent polymer in the side absorbent member to the content ratio of the absorbent polymer in the central absorbent member is from 1.3 to 3, preferably from 1.5 to 2.5.
{10} The disposable diaper as set forth in clause {8} or {9}, wherein the content by percentage of the absorbent polymer in the central absorbent member is from 30% to 70%, more preferably from 35% to 65%.
{11} The disposable diaper as set forth in any one of clauses {8} to {10}, wherein the content by percentage of the absorbent polymer in the side absorbent member is from 40% to 100%, more preferably from 45% to 90%.
{12} The disposable diaper as set forth in any one of clauses {1} to {11}, wherein the absorbent core's basis weight in the side absorbent member is greater than the absorbent core's basis weight in the central absorbent member.
{13} The disposable diaper as set forth in clause {12}, wherein a ratio B2/B1 of B2 to B1 is from 1.1 to 3, preferably from 1.5 to 2.5, where B1 is the absorbent core's basis weight in the central absorbent member and B2 is the absorbent core's basis weight in the side absorbent member.
{14} The disposable diaper as set forth in clause {12} or {13}, wherein the absorbent core's basis weight in the side absorbent member is from 80 to 700 g/m², preferably from 150 to 600 g/m².
{15} The disposable diaper as set forth in any one of clauses {12} to {14}, wherein the absorbent core's basis weight in the central absorbent member is from 1 to 200 g/m², preferably from 5 to 100 g/m².
{16} The disposable diaper as set forth in any one of clauses {1} to {15}, wherein a pair of bending-guide portions, each constituted by a depression, is formed between the central absorbent member and the respective side absorbent members.
{17} The disposable diaper as set forth in any one of clauses {1} to {16}, wherein a pair of bending-guide portions, each constituted by a through hole, is formed between the central absorbent member and the respective side absorbent members.
{18} The disposable diaper as set forth in clause {16} or {17}, wherein the pair of bending-guide portions is formed rectilinearly.
{19} The disposable diaper as set forth in clause {18}, wherein, in the reference swollen state, the absorbent core's width in the central absorbent member is from 8 to 35 mm, preferably from 10 to 30 mm.
{20} The disposable diaper as set forth in clause {18} or {19}, wherein the pair of bending-guide portions is formed curvilinearly.
{21} The disposable diaper as set forth in claim 20, wherein the absorbent core in the central absorbent member has a narrowed portion where both lateral side edges thereof extending along the longitudinal direction are narrowed inwardly in the width direction, the narrowed portion being located more toward the front portion side than the center in the longitudinal direction.
{22} The disposable diaper as set forth in clause {21}, wherein the narrowed portion is located in the crotch portion.
{23} The disposable diaper as set forth in clause {21}, wherein the narrowed portion is located more toward the front portion side than the center of the crotch portion in the longitudinal direction.
{24} The disposable diaper as set forth in any one of clauses {20} to {23}, wherein:
   the width of each bending-guide portion varies along the longitudinal direction; and
   each bending-guide portion has a maximum width portion where the width becomes the greatest, the maximum width portion being located more toward the front portion side than the center in the longitudinal direction.
{25} The disposable diaper as set forth in clause {24}, wherein, in a state before the absorbent core absorbs liquid, the maximum width portion's width is from 10 to 30 mm, preferably from 12 to 20 mm.
{26} The disposable diaper as set forth in any one of clauses {1} to {25}, wherein the central absorbent member includes a non-fiber-stack portion where there is no material forming the absorbent core.
{27} The disposable diaper as set forth in any one of clauses {1} to {26}, wherein:
   in the reference swollen state, compression load to compress the side absorbent member until the side absorbent member's maximum thickness becomes 10 mm is 4.5 N or less; and
   in the reference swollen state, the side absorbent member's maximum thickness is from 10 to 30 mm.
{28} The disposable diaper as set forth in any one of clauses {1} to {27}, wherein the absorbent core has a narrowed portion where the absorbent core's both lateral side edges extending along the longitudinal direction are narrowed inwardly in the width direction, the narrowed portion being located more toward the front portion side than the center in the longitudinal direction.

### Industrial Applicability

With the disposable diaper of the present invention, the wearer's movement around the femoral region is less likely to be restricted, even when the absorbent member has absorbed liquid and has swollen, thereby making it easy to walk in.

## Claims

1. A disposable diaper comprising a topsheet, a backsheet, and an absorbent member arranged between the topsheet and the backsheet,
the absorbent member including an absorbent core,
the disposable diaper having a longitudinal direction and a width direction orthogonal to the longitudinal direction,
the disposable diaper having a rear portion to be arranged on a wearer's rear side when worn, a front portion to be arranged on the wearer's front side when worn, and a crotch portion located between the front portion and the rear portion, wherein:
in the crotch portion, the absorbent member includes
a central absorbent member extending in the longitudinal direction, and
a pair of side absorbent members respectively located on both widthwise sides of the central absorbent member, the side absorbent members being configured to stand up from a skin-facing surface side of the central absorbent member in a worn state;
in a reference swollen state achieved by injecting 160 g of artificial urine to an injection point located 7 cm away toward the front portion side in the longitudinal direction from a center position of the disposable diaper, which is at a center in the longitudinal direction and at a center in the width direction, and leaving the disposable diaper to stand for 5 minutes, compression load to compress a test piece of the disposable diaper until the test piece's thickness becomes 30 mm is 7 N or less in the crotch portion, the test piece being formed by folding the disposable diaper along the longitudinal direction in a manner that the disposable diaper's width is divided equally in two; and
in the reference swollen state, the side absorbent member's thickness is 10 mm or less, or compression load to compress the side absorbent member until the thickness thereof becomes 10 mm is 4.5 N or less.

2. The disposable diaper according to claim 1, wherein, in a state before the absorbent member absorbs liquid, the side absorbent member's thickness is greater than the central absorbent member's thickness.

3. The disposable diaper according to claim 1 or 2, wherein:
the absorbent core includes an absorbent polymer; and
the side absorbent member has a higher content ratio of the absorbent polymer than the central absorbent member.

4. The disposable diaper according to any one of claims 1 to 3, wherein the absorbent core's basis weight in the side absorbent member is greater than the absorbent core's basis weight in the central absorbent member.

5. The disposable diaper according to any one of claims 1 to 4, wherein a ratio B2B 1 of B2 to B1 is from 1.1 to 3, where B1 is the absorbent core's basis weight in the central absorbent member and B2 is the absorbent core's basis weight in the side absorbent member.

6. The disposable diaper according to any one of claims 1 to 5, wherein the absorbent core's basis weight in the side absorbent member is from 80 to 700 g/m².

7. The disposable diaper according to any one of claims 1 to 6, wherein the absorbent core's basis weight in the central absorbent member is from 1 to 200 g/m².

8. The disposable diaper according to any one of claims 1 to 7, wherein a pair of bending-guide portions, each constituted by a depression, is formed between the central absorbent member and the respective side absorbent members.

9. The disposable diaper according to any one of claims 1 to 8, wherein a pair of bending-guide portions, each constituted by a through hole, is formed between the central absorbent member and the respective side absorbent members.

10. The disposable diaper according to claim 8 or 9, wherein the pair of bending-guide portions is formed rectilinearly.

11. The disposable diaper according to claim 8 or 9, wherein the pair of bending-guide portions is formed curvilinearly.

12. The disposable diaper according to claim 11, wherein the absorbent core in the central absorbent member has a narrowed portion where both lateral side edges thereof extending along the longitudinal direction are narrowed inwardly in the width direction, the narrowed portion being located more toward the front portion side than the center in the longitudinal direction.

13. The disposable diaper according to claim 11 or 12, wherein:
the width of each of the bending-guide portions varies along the longitudinal direction; and
each of the bending-guide portions has a maximum width portion where the width becomes the greatest, the maximum width portion being located more toward the front portion side than the center in the longitudinal direction.

14. The disposable diaper according to any one of claims 1 to 13, wherein the central absorbent member includes a non-fiber-stack portion where there is no material forming the absorbent core.

15. The disposable diaper according to any one of claims 1 to 14, wherein:
in the reference swollen state, compression load to compress the side absorbent member until the side absorbent member's thickness becomes 10 mm is 4.5 N or less; and
in the reference swollen state, the side absorbent member's thickness is from 10 to 30 mm.

16. The disposable diaper according to any one of claims 1 to 15, wherein the absorbent core has a narrowed portion where the absorbent core's both lateral side edges extending along the longitudinal direction are narrowed inwardly in the width direction, the narrowed portion being located more toward the front portion side than the center in the longitudinal direction.
